Europäisches Patentamt

European Patent Office

Office européen des brevets ·

(19)

(11) Publication number: **0 068 725**
**B1**

# EUROPEAN PATENT SPECIFICATION

(12)

(45) Date of publication of patent specification: **19.02.86**

(51) Int. Cl.⁴: **C 07 C 45/00, C 07 C 47/565**

(21) Application number: **82303130.7**

(22) Date of filing: **16.06.82**

(54) **Process for preparation of hydroxybenzaldehydes.**

(30) Priority: **16.06.81 JP 93314/81**

(43) Date of publication of application:
**05.01.83 Bulletin 83/01**

(45) Publication of the grant of the patent:
**19.02.86 Bulletin 86/08**

(84) Designated Contracting States:
**CH DE FR GB LI NL**

(56) References cited:
**US-A-3 206 513**
**US-A-3 972 945**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Hamada, Kazuhiko**
**No. 2-1-248, Kuwatacho Ibarakishi**
**Osaka-shi Osaka (JP)**
Inventor: **Suzukamo, Gohfu**
**No. 2-1-216, Kuwatacho Ibarakishi**
**Osaka-shi Osaka (JP)**

(74) Representative: **Diamond, Bryan Clive et al**
**Gee & Co. Chancery House Chancery Lane**
**London WC2A 1QU (GB)**

Courier Press, Leamington Spa, England.

## Description

The present invention relates to an improved process for the preparation of hydroxybenzaldehydes, particularly, salicylaldehyde, or mixtures thereof.

Salicylaldehyde (o-hydroxy benzaldehyde) is an industrially important compound as an intermediate chemical, e.g. for the preparation of perfumes, pesticides or chelating agents.

The following methods are known for the preparation of hydroxybenzaldehydes:

(1) The Reimer-Tiemann Reaction:

In this reaction, a phenol is reacted in a physically heterogenous system with chloroform and an aqueous alkali (such as an alkali metal hydroxide) in aqueous solution, to prepare a mixture of salicylaldehyde and p-hydroxybenzaldehyde. Generally, in this method the yield of salicylaldehyde is low. Further, an excess amount of chloroform over the phenol is necessary, and recovery and recycling of unreacted chloroform and phenol are not necessarily easy (and are expensive).

The Dow method is an example of an improved way of performing the Reimer-Tiemann reaction; a phenol, chloroform and an alkali metal hydroxide are reacted in a solvent medium consisting of aqueous methanol containing 10 to 75% by weight of methanol (see U.S. Patents 3,206,513 and 3,365,500). Even in this method, the conversion of the phenol is low, and the separation and recovery of unreacted phenol are difficult. Further, the yield ratio of salicylaldehyde and p-hydroxybenzaldehyde is lower than that obtained under the ordinary Reimer-Tiemann conditions and there is difficulty in the separation of these products from the reaction mixture.

(2) Method proposed by Yoel Sasson et al [Tetrahedron Lett., 3753 (1979) and 1875 (1980)]:

In accordance with this method, when an aliphatic tertiary amine is added to the reaction mixture obtained under the ordinary Reimer-Tiemann condition (where excess amounts of chloroform and an aqueous alkali solution are used with respect to the phenol), the yield of salicyclaldehyde is improved without any undesirable effect on the yield of p-hydroxybenzaldehyde. But this method has the following disadvantages. Firstly, only a limited number of tertiary amines can be used, such as $(n\text{-}C_4H_9)_3N$, and a reaction to form an alkyl ether of phenol also takes place competitively. Further, excess amounts of chloroform and an aqueous alkali solution over the phenol need be used, which leads to the foregoing disadvantages. Still further, it has been experimentally confirmed by the present inventors under the same conditions as reported that the effects brought by the addition of the aliphatic tertiary amine are not as beneficial as reported.

(3) A method in which a salicylaldehyde is prepared in good yield by one-step reaction using as starting materials a phenol and a formaldehyde and a specific catalyst (a base presented by a tertiary amine and/or an organometallic salt):

The method is described in the published unexamined Japanese Patent Applications (OPI) Nos 34737/78 and 163538/79 and Chem. Soc., Perkin I, p. 1862 (1980). This method, however, also has disadvantages; an excess amount of formaldehyde over the phenol, a poisonous catalyst (e.g., $SnCl_2$, $SnCl_4$ or $Cr(acac)_3$) or a fairly large amount of an organic amine as an additive need be used, and therefore, a complicated post-treatment and waste water treatment and the like are needed.

A method for the selective synthesis of salicylaldehyde by reacting a phenol with an alkylene hydroxide and chloroform and catalysed by means of an N,N-dimethyl formamide or acetamide or dimethyl sulfoxide is described in U.S. Patent No. 3,972,945 (1976). Water is formed during the reaction so that the system is not completely anhydrous, but the water formed is reacted with excess sodium hydroxide. There is negligible production of the corresponding 4-hydroxybenzaldehyde.

In the present process a different type of catalyst is used, namely a surfactant.

According to the present invention we provide a process for the preparation of a mixture of salicylaldehyde and p-hydroxybenzaldehyde, characterised by reacting (a) an alkali metal salt of a phenol, which is not formed in situ from a phenol and an alkali metal hydroxide, (b) chloroform and (c) a slurry of powdered alkali metal hydroxide and an anhydrous inert organic liquid, using a catalytic amount of a surfactant under an anhydrous or substantially anhydrous reaction condition.

By this process the desired aldehyde is prepared selectively and in good yield without the use of poisonous or unpleasant catalysts and without need for post-treatment. The alkali metal phenolate can easily be prepared in advance.

It has been found that the o- or p-hydroxybenzaldehyde can thus be prepared selectively without decomposition of the other hydroxybenzaldehyde, in a reaction system where the reaction is thus initiated under an anhydrous or substantially anhydrous condition.

It is generally agreed that in the traditional Reimer-Tiemann reaction excess of an aqueous alkaline solution over the phenol should be used. In contrast, in the process of the invention, an alkali phenolate is reacted with chloroform under heating in a slurry of a powdered alkali metal hydroxide individually prepared in advance under anhydrous or substantially anhydrous condition; this is a new way of carrying out the Reimer-Tiemann reaction. Consequently, it is quite unexpected that salicylaldehyde can be prepared with good yield and high selectivity under the conditions specified in the present invention.

Examples of alkali phenolates as starting material which can be used in the invention are lithium phenolate, sodium phenolate, potassium phenolate and cesium phenolate. Of these compounds, sodium phenolate and potassium phenolate are preferable because these materials are inexpensively available.

The alkali phenolates which are used as the starting material can be, for example, prepared by one of the following methods. (1) After dissolving phenol in dried toluene, an equimolar amount of an alkali metal (e.g., commercially available metallic sodium or metallic potassium) is added to the solution, and the mixture is heated, followed by concentrating a toluene solution containing the alkali metal phenolate and then drying it under reduced pressure (see *Organic Synthesis, Coll.,* Vol. 1, p. 58). (2) An aqueous solution of 50 weight % sodium hydroxide is added to phenol, and the mixture is stirred and heated. Water present in the reaction system is then removed, and the residue is concentrated and dried under reduced pressure. In this method (2), the water content of the alkali metal phenolate used as the starting material should be less than 3 to 5 weight %.

The slurry of alkali metal hydroxide in organic solvent can be prepared in the following method. An alkali metal hydroxide (e.g., sodium hydroxide, potassium hydroxide or lithium hydroxide) is added to an organic liquid which is inert to the reaction and is a solvent for the phenolate and chloroform, and the wet mixture is intensely shaken to thereby form a thick suspension containing particles of 30 to 60 mesh size (100 to 700 µm particle size). Examples of the solvent used are aromatic hydrocarbons (e.g., benzene, toluene, xylene), ethers (e.g., anisol, dibutyl ether, diisopropyl ether), esters (e.g., ethyl acetate, methyl acetate) and aliphatic alcohols (e.g., methanol, ethanol, butanol, ethylene glycol). It is possible, to some extent, to control the selectivity of the product by selecting the solvent used. Powdered sodium or potassium hydroxide in an aromatic hydrocarbon such as benzene or toluene is preferred. The weight ratio of hydroxide to solvent is from 0.05:1 to 2:1, preferably 0.01:1 to 0.5:1.

The slurry can be prepared as follows. Pellets of sodium hydroxide having a purity higher than 95 weight % and some definite excess amount of dried (anhydrous) benzene or toluene over the hydroxide are charged into a vessel made of polyethylene. Alumina balls (diameter 5 to 10 mm, 10 to 15 balls per 20 to 25 g of slurry) are added thereto, then the system is shielded, fastened firmly and shaken intensely in a wet phase by means of a paint shaker for 30 minutes to 1 hour, to thereby obtain a desired slurry. When pellet-type potassium hydroxide having a purity higher than 85 weight % is used, it is preliminarily melted by heating and dehydrated by drying under reduced pressure to prepare solid potassium hydroxide. Some definite excess amount of a dried organic solvent over the potassium hydroxide is added thereto, and the mixture is shaken intensely in a wet phase as described above for sodium hydroxide, to obtain a slurry. The alkali concentration of the thus-obtained slurry is determined from the relation between the amount of the anhydrous state alkali hydroxide used and the amount of the excess of organic solvent used.

The chloroform used can be normal grade reagent without any pretreatment.

It is essential to add to the reaction system a surfactant in a catalytic amount; this improves the conversion of the alkali phenolate and the ratio of formation of hydroxybenzaldehyde. Surfactants which can be used include nonionic, cationic, anionic and amphoteric surfactants. They can be used alone or in combination. A preferable amount of surfactant is from 0.1 to 5.0 mol % of the alkali phenolate.

Specific examples of suitable surfactants are:

*nonionic*: polyethylene glycol-based surfactants (e.g., higher alcohol, addition compounds formed from (a) a phenol or fatty acid and (b) ethylene oxide) and polyhydric alcohol-based surfactants (e.g., higher fatty acid sorbitan esters or sugar esters),

*cationic*: quaternary ammonium salt-based surfactants (e.g., cetyl trimethylammonium chloride or tetrabutylammonium hydroxide), quaternary phosphonium salt-based surfactants and pyridinium salt based surfactants,

*anionic*: alkali metal salts of higher fatty acids, higher alkyl sulphonic acid salts and sulfuric ester salts of higher alcohols,

*amphoteric*: amino acid-based surfactants and betaine-based surfactants.

Of these, nonionic surfactants are preferred. A sorbitan trioleate polyoxyalkylene derivative or sorbitan trioleate are preferred surfactants to enable the reaction to proceed smoothly.

As for the proportion of the reactants in the reaction system, these are employable within a range of from the ordinary laboratory condition wherein a large excess amount of an alkali and chloroform to phenol (calculated from the alkali phenolate) is employed to not more than the estimated theoretical amount thereof (i.e., phenol/alkali/chloroform = 1.0/4.0/1.0). Generally, the reactants are used in a range of phenol/alkali/chloroform of from 1.0/10.0/10.0 to 1.0/2.0/0.3.

The reaction according to the present invention can be carried out in the following manner. Predetermined amounts of an alkali phenolate, the slurry of powdered alkali metal metal hydroxie in inert organic solvent and optionally, a surfactant are charged into a reactor equipped with a condenser, and a predetermined amount of chloroform is added to the stirred mixture under heating (generally, at a temperature of from 50°C to 100°C), whereby the reaction commences, and is substantially completed within 30 minutes to 10 hours. After completion of the reaction, the reaction mixture is cooled to room temperature (e.g., 15° to 30°C), and a predetermined amount of ethyl acetate or methyl isobutyl ketone is added thereto. Thereafter, an excess amount of concentrated hydrochloric acid (12 to 34 weight %) is added to the resulting mixture under cooling and stirring to adjust the pH of the aqueous acid layer to 1 to 2. After the separation of the organic layer, the aqueous layer is extracted twice with ethyl acetate, and the extracts are collected together. Analysis of the product can be carried out by analyzing the organic layer by a usual method such as gas chromatography (internal standard method).

The invention is illustrated by the following Examples.

Example 1

A three-necked flask equipped with a condenser and a stirrer was charged with 3.0 g (0.0259 mol) of sodium phenolate and 20.66 g of a slurry of powdered sodium hydroxide in anhydrous benzene [NaOH particles 100 to 700 μm size 5.16 g (0.129 mol), and benzene 15.5 g], each of which was individually prepared in advance, and 0.15 g (0.00014 mol) of a sorbitan monooleate polyoxyalkylene derivative sold under the trade name "Tween 80". The mixture was heated to 60 to 65°C and stirred at 150 to 300 rpm. When the temperature of the system became constant, 18.5 g (0.155 mol) of chloroform charged by the side inlet tube was carefully added dropwise to the stirred mixture over a period of 30 minutes to 1 hour. After the completion of dropwise addition, the reaction temperature was elevated to 75 to 80°C, at which temperature the mixture was held and stirred for about a further 3 hours. The initiation of the reaction could be visually observed by the change of color of the emulsion to yellowish brown and the dropwise addition of chloroform commenced.

After the completion of reaction, the reaction mixture was cooled to room temperature (i.e., 15 to 30°C), and 10 ml of ethyl acetate (or methyl isobutyl ketone) was added thereto. Thereafter, concentrated hydrochloric acid (12 weight %) in an amount of 2 to 3 times the stoichiometric amount was added to the reaction mixture under cooling to 0 to 5°C, and the resulting mixture was stirred to adjust the pH of the aqueous layer to 1 to 2. After the separation of the ethyl acetate layer, the aqueous layer was thoroughly extracted twice with 10 ml portions of ethyl acetate. These extracts were collected together, and then subjected to quantitative analysis by gas chromatography. As the result, there was found a conversion based on sodium phenolate used of the phenol of 95.7% and a yield, based on phenol. consumed, of hydroxybenzaldehydes (salicylaldehyde plus p-hydroxybenzaldehyde) of 62.8%. The proportion of salicylaldehyde to p-hydroxybenzaldehyde formed was 7.8:1.

Examples 1 to 9 and Controls 1 and 2

The reaction was carried out in the same manner as in Example 1. The results obtained are shown in Table 1 below. In Table 1, SAL and POBA mean salicylaldehyde and p-hydroxybenzaldehyde, respectively. "Span 85" is the trade name of a sorbitan monooleate surfactant.

TABLE 1

| Example No. | Reactant Base | Anhydrous Alkali/Organic Solvent Slurry | Chloroform (to be added dropwise) | Proportion of ($C_6H_5OH^*$/MOH/$CHCl_3$) Charged | Surfactant (mol % of alkali phenolate) | Reaction Condition Temperature (°C) | Time (hr) | Conversion of alkali phenolate (%) | Yield of SAL+POBA (based on alkali phenolate consumed) (%) | SAL/POBA |
|---|---|---|---|---|---|---|---|---|---|---|
| Control 1 | $C_6H_5ONa$ (3.0 g) | NaOH / $C_6H_6$ | $CHCl_3$ | 0.17/1.0/1.0 | Nil | 75–80 | 5.0 | 65.0 | 30.0 | 5.7 |
| Control 2 | ,, | NaOH / toluene | $CHCl_3$ | 0.17/1.0/1.0 | Tween 80 (0.54) | 80–85 | 4.5 | 88.9 | 60.1 | 6.9 |
| Control 3 | ,, | NaOH / $Bu_2O$ | $CHCl_3$ | 0.17/1.0/1.0 | Tween 80 (0.54) | 80–85 | 4.5 | 80.3 | 59.3 | 6.6 |
| Control 4 | $C_6H_5OK$ (3.0 g) | KOH / $C_6H_6$ | $CHCl_3$ | 0.17/1.0/1.0 | Tween 80 (0.54) | 75–80 | 4.5 | 70.8 | 46.5 | 4.5 |
| Control 5 | $C_6H_5ON$ (3.0 g) | NaOH / $C_6H_6$ | $CHCl_3$ | 0.17/1.0/1.0 | Span 85 (0.50) | 75–80 | 4.5 | 82.7 | 53.7 | 5.13 |
| Control 6 | ,, | ,, | $CHCl_3$ | 0.17/1.0/1.0 | $C_{12}H_{25}NMe_3Cl$ (1.0) | 75–80 | 4.5 | 92.1 | 61.0 | 7.4 |

TABLE 1 (Continued)

| Example No. | Reactant Base | Anhydrous Alkali/Organic Solvent Slurry | Chloroform (to be added dropwise) | Proportion of OH*/MOH/CHCl$_3$ Charged | Surfactant (mol % of alkali phenolate) | Reaction Condition Temperature (°C) | Time (hr) | Conversion of alkali phenolate (%) | Yield of SAL+POBA (based on alkali phenolate consumed) (%) | SAL/POBA |
|---|---|---|---|---|---|---|---|---|---|---|
| Control 7 | OLi (3.0 g) | LiOH/ | CHCl$_3$ | 0.17/1.0/1.0 | Tween 80 (0.70) | 85—90 | 5.5 | 75.7 | 53.9 | 8.4 |
| Control 8 | ONa (3.0 g) | NaOH/ | CHCl$_3$ | 3.0/6.0/1.0 | Tween 80 (0.50) | 60—65 | 6.5 | 34.2 | 58.0 | 5.2 |
| Control 9 | ,, | ,, | CHCl$_3$ | 1.5/5.4/1.0 | Tween 80 (0.50) | 75—80 | 5.0 | 48.9 | 61.3 | 7.0 |
| Control 2 | OK (3.0 g) | KOH/ HOCH$_2$CH$_2$OH | CHCl$_3$ | 0.17/1.0/1.0 | Nil | 80—85 | 4.5 | 39.7 | 73.1 | 0.77 |

*: Calculated from alkali phenolate.

## Claims

1. A process for the preparation of a mixture of salicylaldehyde and p-hydroxybenzaldehyde, characterized by reacting an alkali metal salt of a phenol, which is not formed in situ from a phenol and an alkali metal hydroxide, chloroform and a slurry of a powdered alkali metal hydroxide and an anhydrous inert organic liquid, using a catalytic amount of a surfactant under an anhydrous or substantially anhydrous reaction condition.

2. A process as claimed in Claim 1, wherein the alkali metal hydroxide is in the form of a suspended fine powder in said slurry.

3. A process as claimed in Claim 1 or 2, wherein said alkali metal hydroxide is sodium hydroxide and/or potassium hydroxide.

4. A process as claimed in Claim 1, 2 or 3, wherein the organic liquid is an aromatic hydrocarbon, an ester or an aliphatic alcohol.

5. A process as claimed in any of Claims 1 to 4, wherein the amount of the surfactant is from 0.1 to 5.0 mol% of the alkali metal phenolate.

6. A process as claimed in any of Claims 1 to 5, wherein the reactants are used in a stoichiometric ratio of the phenol moiety of the phenolate to alkali metal hydroxide to chloroform of from 1.0:10.0:10.0 to 1.0:2.0:0.3.

7. A process as claimed in any of Claims 1 to 6, wherein the weight ratio of alkali metal hydroxide to organic solvent is from 0.05:1 to 2:1.

8. A process as claimed in any of Claims 1 to 7, wherein the reaction is carried out whilst stirring the reaction mixture at a speed of 150 to 300 revolutions per minute.

## Patentansprüche

1. Verfahren zur Herstellung eines Gemisches von Salicylaldehyd und p-Hydroxybenzaldehyd, gekennzeichnet durch die Umsetzung eines Alkalimetallphenolats, das nicht in situ aus einem Phenol und einem Alkalimetallhydroxid hergestellt wird, mit Chloroform und einer Paste eines pulverförmigen Alkalimetallhydroxids und einer wasserfreien organischen inerten Flüssigkeit unter Verwendung einer katalytischen Menge eines grenzfächenaktiven Mittels unter wasserfreien oder praktisch wasserfreien Bedingungen.

2. Verfahren nach Anspruch 1, gekennzeichnet durch die Verwendung des Alkalimetallhydroxids in Form eines in der Paste suspendierten feinen Pulvers.

3. Verfahren nach Anspruch 1 oder 2, gekennzeichnet durch die Verwendung von Natriumhydroxid und/oder Kaliumhydroxid als Alkalimetallhydroxid.

4. Verfahren nach Anspruch 1, 2 oder 3, gekennzeichnet durch die Verwendung eines aromatischen Kohlenwasserstoffs, Esters oder aliphatischen Alkohols als organische Flüssigkeit.

5. Verfahren nach einem der Ansprüche 1 bis 4, gekennzeichnet durch die Verwendung des grenzflächenaktiven Mittels in einer Menge von 0,1 bis 5,0 mol-%, bezogen auf das Alkalimetallphenolat.

6. Verfahren nach einem der Ansprüche 1 bis 5, gekennzeichnet durch die Verwendung der Reaktionsteilnehmer in einem stöchiometrischen Verhältnis von Phenolkern zu Phenolat zu Alkalimetallhydroxid zu Chloroform von 1,0:10,0:10,0 bis 1,0:2,0:0,3.

7. Verfahren nach einem der Ansprüche 1 bis 6, gekennzeichnet durch die Verwendung des Alkalimetallhydroxids und des organischen Lösungsmittels in einem Gewichtsverhältnis von 0,05:1 bis 2:1.

8. Verfahren nach einem der Ansprüche 1 bis 7, gekennzeichnet durch die Durchführung der Umsetzung unter Rühren des Reaktionsgemisches mit einer Geschwindigkeit von 150 bis 300 Umdrehungen/min.

## Revendications

1. Procédé pour la préparation d'un mélange de salicylaldéhyde et de p-hydroxybenzaldéhyde, caractérisé par le fait qu'on fait réagir un selde métal alcalin d'un phénol, qui n'est pas formé in situ à partir d'un phénol et d'un hydroxyde de métal alcalin, du chloroforme et une pâte d'un hydroxyde de métal alcalin poudreux et d'un liquide organique inerte anhydre, en utilisant une quantité catalytique d'un tensio-actif dans des conditions de réaction anhydre ou substantiellement anhydre.

2. Procédé selon la revendication 1, dans lequel l'hydroxyde de métal alcalin est sous la forme d'une poudre fine suspendue dans ladite pâte.

3. Procédé selon la revendication 1 ou la revendication 2, dans laquelle ledit hydroxyde de métal alcalin est l'hydroxyde de sodium et/ou l'hydroxyde de potassium.

4. Procédé selon la revendication 1, 2, ou 3, dans lequel le liquide organique est un hydrocarbure aromatique, un ester ou un alcool aliphatique.

5. Procédé selon l'une des revendications 1 à 4, dans lequel la quantité de tensio-actif est comprise entre 0,1 et 5,0 mole % du phénolate de métal alcalin.

6. Procédé selon l'une des revendications 1 à 5, dans lequel les réactifs sont utilisés en un rapport stoichiométrique du noyau phénol au phénolate à l'hydroxyde de métal alcalin et au chloroforme compris entre 1,0:10,0:10,0 à 1,0:2,0:0,3.

7. Procédé selon l'une des revendications 1 à 6, dans lequel le rapport en poids de l'hydroxyde de métal alcalin au solvant organique est compris entre 0,05:1 et 2:1.

8. Procédé selon l'une des revendications 1 à 7, dans lequel la réaction est effectuée en agitant le mélange réactionnel à une vitesse de 150 à 300 tours par minutes.